# EUROPEAN PATENT APPLICATION

(11) **EP 4 837 657 A1**
(43) Date of publication of application: **11.11.2026**
(21) Application number: 25175095.6
(22) Date of filing: 08.05.2025
(51) Int. Cl.: B03C 3/155, B03C 3/41, B03C 3/68

(54) **AIR TREATEMENT SYSTEM**

(71) Applicant: Wood's Production Sweden AB, 441 39 Alingsås (SE)
(72) Inventor: Tedsjö, Patrik, 441 39 Alingsås (SE)
(74) Representative: Valea AB

(57) **Abstract**

This disclosure relates to an air treatment system (100) comprising an electrostatic charging unit (101) comprising at least one conductive element (104) arranged to apply surface charge to airborne particles in an airflow passing through the electrostatic charging unit (101). The air treatment system (100) comprises an air filter (103) arranged downstream of the electrostatic charging unit (101) arranged to filter the airflow from the electrostatic charging unit (101).

## Description

### TECHNICAL FIELD

The present disclosure relates generally to an air treatment system.

### BACKGROUND

Air treatment systems are commonly used in various applications, aimed at improving indoor air quality. These devices include a variety of technologies such as air purifiers, humidifiers, dehumidifiers, and air conditioning systems. Each type of device serves a specific purpose in air quality management, contributing to the overall health and comfort of occupants.

Air treatment systems such as air purification systems, also referred to as air filtration systems, are commonly used in indoor locations for improving air quality. Indoor air quality is increasingly recognized as a factor affecting human health and well-being. Poor indoor air quality may lead to various health issues, including respiratory problems, allergies, and overall discomfort. The sources of indoor air pollution are diverse, including dust, pollen, pet dander, mold spores, volatile organic compounds emitted from household products, and even pathogens. As people spend a significant portion of their time indoors, effective air treatment systems have become essential for creating healthier indoor environments.

Traditional air purification systems often rely on passive mechanical filtration or fixed ionizers to capture airborne particles. Both passive mechanical filters and fixed ionizers may exhibit inconsistent performance, particularly as they become loaded with particulates or if they are not maintained properly. Traditional air purification systems often lack the ability to adapt to changing indoor air quality conditions. For example, they may not increase filtration rates in response to higher levels of pollutants or allergens. Many users may be unaware of the need for regular maintenance and filter changes, leading to suboptimal performance over time.

While traditional air purification systems using passive mechanical filtration or fixed ionizers have been widely used, their limitations highlight the need for more advanced solutions that enhance filtration efficiency, reduce maintenance requirements, and improve adaptability to varying indoor air quality conditions.

Therefore, there is a need to at least mitigate or solve this issue.

### SUMMARY

An objective is to mitigate or solve at least one of the issues mentioned above and to provide an improved air treatment system.

According to a first aspect, the objective is achieved by an air treatment system. The air treatment system comprises an electrostatic charging unit comprising at least one conductive element arranged to apply surface charge to airborne particles in an airflow passing through the electrostatic charging unit. The air treatment system comprises an air filter arranged downstream of the electrostatic charging unit arranged to filter the airflow from the electrostatic charging unit.

An advantage may be that filter efficiency is enhanced. The electrostatic charging unit applies a surface charge to airborne particles, and this charge makes it easier for the air filter to capture these particles, including smaller ones that might otherwise pass through. This means cleaner air for indoor environments. Another advantage may be that particle attraction is improved. By charging the particles, the air treatment system increases its attraction to the filter media. This helps to gather more contaminants, such as dust, pollen, and even some pathogens, enhancing overall air quality. A further advantage may be that pressure drop is reduced. With effective charging, the air filter may work more efficiently, potentially leading to a lower pressure drop across the air filter. This means that the air treatment system may maintain good airflow while effectively trapping contaminants, which is crucial for energy efficiency. Yet another advantage may be that the air filter life may be extended. Because the electrostatic charging helps to capture particles more effectively, the air filter may not clog as quickly. This may extend the life of the air filter, reducing the frequency of replacements and saving on maintenance costs. Another advantage may be that versatility in air quality management may be obtained. The air treatment system may adapt to different air quality needs. By adjusting the electrostatic charging, it may be tuned to target specific airborne particles, making it versatile for various indoor environments, such as homes, offices, or industrial spaces. Yet another advantage may be that the performance in challenging conditions may be improved. In environments with high levels of airborne particles, this air treatment system may still function effectively. The electrostatic charging helps to maintain filtration efficiency even when particle loads are high, ensuring consistent air quality. Another advantage may be that it may enable a potential for smart integration. The design of the air treatment system allows for the potential integration of smart sensors that can monitor air quality in real-time. This means the air treatment system mat adjust the electrostatic charge based on current air quality conditions, optimizing performance dynamically. In summary, the air treatment system improves air quality, lowers maintenance costs, and enhances performance in various indoor settings.

The at least one conductive element may be arranged transverse to the direction of airflow through the air treatment system. The at least one conductive element may be arranged on a support structure. An advantage may be that the particle charging is improved. When the conductive element is placed across the airflow, it may effectively charge more airborne particles. This means that more particles get an electrostatic charge, making them easier to capture later. Another advantage may be that the surface area is increased. The transverse arrangement may maximize the surface area that particles may come into contact with. The more area available, the better the chances of charging the particles effectively. Yet another advantage may be that the airflow dynamics may be improved. Positioning the conductive element this way helps maintain smoother airflow. This may reduce turbulence, which may improve overall efficiency and performance of the air treatment system. Another advantage may be that a more uniform distribution of charge may be provided. Having the conductive element transverse may allow for a more even distribution of charge in the air. This may help ensure that a variety of particle sizes are effectively charged and may be filtered out. A further advantage may be that air filter performance may be enhanced. With better charging and smoother airflow, the system's air filter may work more efficiently. This means it may capture more particles and provide cleaner air for indoor environments. Another advantage may be that the air filter life may be increased. When the air treatment system charges particles effectively, it reduces the load on the air filter. This may lead to less clogging and extend the life of the air filter, saving on maintenance costs. In summary, arranging the conductive element transverse to the airflow boosts the air treatment system's performance. It helps charge particles better, improves airflow, and enhances overall efficiency, leading to cleaner air with less hassle.

The at least one conductive element may be a flexible element. An advantage may be that the contact with the airflow may be improved. A flexible element may easily adjust to changes in the airflow path. This may ensure better contact with airborne particles, enhancing the charging process. Another advantage may be related to adaptability. Flexible conductive elements may conform to different shapes and sizes of the air treatment system. This adaptability may allow for easier integration into various designs, making the system more versatile. A further advantage may be that efficiency of the air treatment may be enhanced. The ability to bend and flex may mean that the conductive element may maintain effective charging even in non-ideal conditions. This may lead to more efficient particle capture and improved air quality. Another advantage may be that breakage may be reduced. Flexible materials are less likely to break or become damaged compared to rigid components. This may lead to a longer lifespan for the conductive element and the overall air treatment system. Another advantage may be that installation and maintenance may be simplified. Flexible elements may be easier to install and replace. Their design may simplify maintenance tasks, making it more user-friendly for operators. In summary, using a flexible conductive element in the air treatment system improves efficiency, adaptability, and durability. It makes the air treatment system more effective at cleaning the air while being easier to maintain and install.

The at least one conductive element may be at least one of: a brush-like element and a fiber-like element. An advantage may be that it may provide effective particle charging. Both brush-like and fiber-like elements have a large surface area. This may help them charge more airborne particles, improving the air treatment system's ability to capture contaminants. Another advantage may be related to increased flexibility. The brush-like element and a fiber-like element may easily bend and move with the airflow. This flexibility allows them to maintain good contact with particles, enhancing their effectiveness in charging. A further advantage may be related to versatile design. Brush-like and fiber-like elements may fit into various designs and configurations. This versatility makes it easier to integrate them into different air treatment systems. Another advantage may be that the airflow may be improved. The structure of these elements may help maintain smooth airflow. They may create less resistance compared to solid conductive surfaces, which helps the system run efficiently. A further advantage may be that the longevity may be enhanced. The materials used for brush-like and fiber-like elements are often durable. This may mean they may withstand wear and tear, leading to a longer lifespan for the conductive components. An advantage may be that the maintenance may be easier. Brush-like and fiber-like elements may be easier to clean or replace than solid conductive components. This makes maintenance simpler and more user-friendly. In summary, using brush-like and fiber-like conductive elements may boost the air treatment system's performance. They enhance particle charging, maintain airflow, and provide flexibility, making the system more effective and easier to maintain.

The electrostatic charging unit may be arranged to be electrically connected to a power supply from which the electrostatic charging unit obtains power. An advantage may be that it provides consistent and reliable performance. Connecting the electrostatic charging unit to a power supply ensures that it receives a steady power. This consistent voltage may be important for maintaining the electrostatic charge on airborne particles, which enhances their attraction to the filter. As a result, the air treatment system operates more efficiently, capturing more contaminants and improving overall air quality. Another advantage may be that the efficiency may be improved. A reliable power source may allow the electrostatic charging unit to function optimally, which means it can effectively charge particles of various sizes. This may lead to better filtration performance, as more particles can be captured by the air filter downstream. A further advantage may be that the control of the air treatment system may be enhanced. With a direct connection to a power supply, the charging unit may be designed to adjust power levels as needed. This flexibility enables the air treatment system to respond to changes in air quality, optimizing performance based on real-time conditions. In summary, by ensuring a steady power supply, the electrostatic charging unit enhances the air treatment system's effectiveness, reduces maintenance needs, and contributes to cleaner indoor air.

The at least one conductive element may be distributed across an area corresponding to a cross-section of an airflow channel. An advantage may be that the particle contact may be maximized. By spreading the conductive element across the entire airflow channel, more airborne particles may come into contact with it. This may increase the chances of charging the particles effectively. Another advantage may be that it may provide uniform charge distribution. A well-distributed conductive element may help ensure that the electrostatic charge is applied evenly. This uniformity may improve the overall efficiency of particle capture in the downstream filter. A further advantage may be that the airflow dynamics may be improved. Distributing the conductive element may help maintain smooth airflow throughout the channel. This may reduce turbulence and keeps the air moving efficiently, which is vital for effective filtration. A further advantage may be that it may enhance filtration performance. With more particles being charged and captured, the overall performance of the air treatment system may be improved. This may lead to cleaner air and better air quality in indoor environments. Another advantage may be that it enables a versatile design integration. This arrangement may allow for flexibility in the design of the air treatment system, making it easier to integrate into different setups while still achieving optimal performance. In summary, distributing the conductive element across the airflow channel may enhance the air treatment system's effectiveness. It may improve particle charging, maintains smooth airflow, and ultimately leads to cleaner indoor air.

The filter may comprise at least two filter layers. Each of the at least two filter layers may be made of at least one of, in any sequence or combination of:
- polypropylene,
- polyester,
- acrylic fiber,
- polyamide,
- polyvinylidenefluoride (PVDF) nanofiber,
- Polytetrafluoroethylene (PTFE) fiber,
- carbon fiber,
- bamboo fiber,
- cellulose fiber,
- graphene-based material, and
- photocatalytic coatings.

The filter may comprise at least two filter layers. The at least two filter layers may comprise a combination of a graphene-based material with at least one of:
- carbon fiber
- photocatalytic TiO₂ coating, and
- polyvinylidenefluoride, PVDF nanofiber.

An advantage may be that the filtration efficiency may be enhanced. Using at least two filter layers may allow for better trapping of particles of different sizes. The combination of materials may capture larger particles in one layer while filtering smaller particles in another, leading to cleaner air. Another advantage may be related to the versatility in material properties:
- Polypropylene and Polyester: These materials may be good for general filtration and are cost-effective.
- Acrylic and Polyamide: These materials may provide durability and strength.
- PVDF Nanofiber: This material may be excellent for capturing fine particles and is resistant to chemicals.
- PTFE Fiber: This material have high-temperature resistance and low friction, enhancing airflow.
- Carbon Fiber: This material may help absorb odors and gases.
- Bamboo and Cellulose Fiber: These materials may be eco-friendly options that may provide good filtration.
- Graphene-Based Material: This material may offer exceptional strength and may enhance filtration efficiency.
- Photocatalytic Coatings: This material may break down harmful pollutants using light, improving air quality.

Another advantage may be that the airflow may be improved. At least two filter layers may be designed to minimize resistance while maintaining high filtration efficiency. This may ensure that the airflow remains strong, making the system more energy efficient. Another advantage may be that the filter lifespan may be extended. With layers working together, the filter may handle more particulate load before clogging. This may lead to longer replacement intervals and lower maintenance costs. A further advantage may be the adaptability to different environments. By selecting different materials for each filter layer, the filter may be tailored to specific applications or environments. This adaptability may make it suitable for a range of indoor air quality needs. Another advantage may be that the capture of gaseous pollutants may be enhanced. Certain materials, like carbon fiber and photocatalytic coatings, may target not just particles but also gaseous pollutants, providing a more comprehensive air purification solution. In summary, using at least two filter layers made from various materials enhances filtration efficiency, improves airflow, and extends the filter's lifespan. It may also allow for customization to meet specific air quality needs, making the air treatment system more effective and versatile.

The air treatment system may comprise or be arranged to be connected to a control unit arranged to control charging of the electrostatic charging unit based on input from an air quality sensor. An advantage may be that the performance may be optimized. The control unit may adjust the charging of the electrostatic unit based on real-time air quality data. This may mean the air treatment system can operate at its best, ensuring effective particle capture when air quality is poor. Another advantage may be that energy efficiency is improved. By only charging, when necessary, the air treatment system saves energy. This may reduce electricity costs and makes the air treatment system more environmentally friendly. A further advantage may be that the equipment life may be prolonged. Smart control may help prevent overcharging, which may damage the electrostatic unit over time. This may lead to longer equipment lifespan and less frequent replacements. Yet another advantage may relate to responsive operation. The air treatment system may quickly adapt to changes in air quality. If pollution levels rise, the air treatment system may ramp up its charging to handle the increased load, providing consistent air quality. Another advantage may be that it may provide user convenience. With automatic adjustments, users don't have to manually control the air treatment system. It may operate in the background, allowing for a hassle-free experience. Another advantage may be that the air quality may be improved. The ability to respond to air quality changes ensures that the air treatment system may effectively target pollutants, leading to cleaner, healthier indoor air. In summary, connecting the air treatment system to a control unit may enhance efficiency, save energy, and improve overall air quality. It may make the air treatment system smarter and easier to use, adapting to the user's needs seamlessly.

The control unit may be arranged to determine an indicator indicative of at least one of:
- a need for maintenance of at least one air treatment component,
- a need for replacement of the at least one air treatment component, and
- an operational status of the at least one air treatment component.

An advantage may be that it may enable proactive maintenance. The control unit may detect when maintenance is needed, helping to avoid unexpected breakdowns. This may keep the air treatment system running smoothly and reduces downtime. Another advantage may be that it enables timely replacement. By indicating when an air treatment component needs replacement, the air treatment system may ensure that filters and other air treatment components are changed at the right time. This may help maintain optimal air quality and system performance. A further advantage may be that reliability may be enhanced. Monitoring the operational status of air treatment components helps identify issues before they become serious problems. This increases the overall reliability of the air treatment system. Another advantage may be that it may improve user convenience. With automatic alerts for maintenance and replacements, users don't have to guess when to check or change air treatment components. This may make the air treatment system easier to manage. A further advantage may be that cost efficiency may be increased. By ensuring timely maintenance and replacements, the air treatment system may operate more efficiently, leading to lower operational costs over time. It also helps extend the lifespan of components. Yet another advantage may be that the air quality may be improved. Regular maintenance and timely replacements ensure that the air treatment system performs at its best, which means cleaner air for users. In summary, having a control unit that tracks maintenance needs, replacement schedules, and operational status boosts the air treatment system's reliability and efficiency. It makes the air treatment system easier to manage and ensures cleaner air in indoor environments.

The air filtration system may be modular, and its air treatment components may be replaceable via a quick-connect interface. An advantage may be that replacement may be easy. It is possible to quickly swap out, for example the electrostatic charging unit without complicated tools. This may save time and hassle when maintenance is needed. Another advantage may be that flexibility may be improved. The modular design may make it possible to upgrade or change the electrostatic charging unit as new technologies become available. It may be possible to keep the air treatment system current without replacing the whole setup.

Another advantage may be related to cost efficiency. Instead of replacing the entire air treatment system, it may only be necessary to replace the electrostatic charging. This may reduce costs and waste. Another advantage may be that the maintenance may be simplified. With a quick-connect interface, routine checks and maintenance may become straightforward. The air treatment system may be run smoothly with minimal downtime.

A further advantage may be that the air treatment system may be user-friendly. The design may be intuitive, making it easy to manage. Specialized training to replace or maintain the electrostatic charging unit may not be necessary. Another advantage may be that the performance may be enhanced. A well-maintained electrostatic charging unit may mean better air filtration. Quick and easy replacement may ensure the air treatment system works efficiently at all times. In summary, a modular electrostatic charging unit with a quick-connect interface may offer easy replacement, flexibility for upgrades, cost savings, simplified maintenance, user-friendliness, and improved performance. It may make managing the air treatment system straightforward and efficient.

Thanks to the electrostatic charging and air filtration, an improved air treatment system is provided.

The embodiments herein are not limited to the features and advantages mentioned above. A person skilled in the art will recognize additional features and advantages upon reading the following detailed description.

### BRIEF DESCRIPTION OF THE DRAWING

The embodiments herein will now be further described in more detail by way of example only in the following detailed description by reference to the appended drawing illustrating the embodiments and in which:

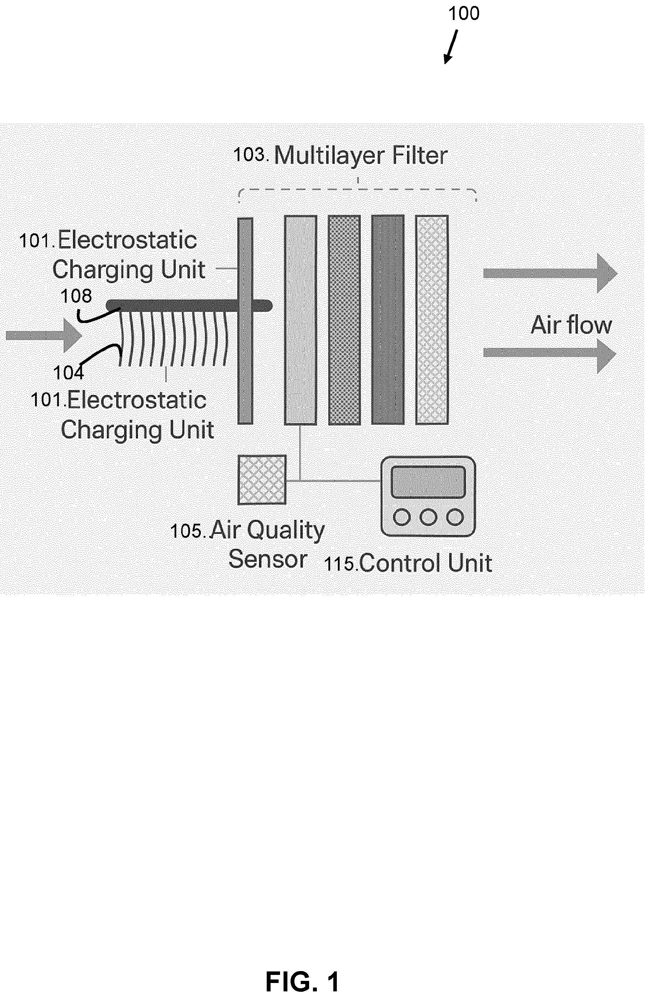

- Fig. 1: is a schematic drawing illustrating an air treatment system.

The drawing is not necessarily to scale and the dimensions of certain features may have been exaggerated for the sake of clarity. Emphasis is instead placed upon illustrating the principle of the embodiments herein.

### DETAILED DESCRIPTION

The present disclosure relates to an air treatment system which may comprise an electrostatic charging unit and multilayer filter. The present disclosure relates to an air treatment system that may utilize flexible brush-like elements, dynamic charging control, and multilayer filters incorporating graphene-based materials.

The present disclosure provides a modular and adaptive air treatment system that may comprise an upstream electrostatic charging unit with conductive flexible elements, and a downstream multilayer filter constructed from selected advanced materials. The air treatment system enhances filtration performance by imparting electrostatic charges to airborne particles, increasing capture efficiency through subsequent filter layers. Materials such as graphene-based media offer additional antimicrobial benefits and surface conductivity. The air treatment system may comprise or may be arranged to be connected to a control unit equipped with smart or Al-driven logic for dynamic response to real-time sensor data, optimizing operational parameters for different environments and usage profiles.

**FIG. 1** is a schematic drawing illustrating an **air treatment system 100.** In short, the air treatment system 100 is arranged to obtain input air, treat the input air and output treated air. Thea air treatment system 100 may be referred to as an air treatment device. The treatment may comprise any suitable treatment, such as for example filtering, purifying, dehumidifying, humidifying, temperature changing etc. The air treatment system 100 may be any suitable device arranged to treat air, for example air purifier, a dehumidifier, a humidifier and an air cooler. The air treatment system 100 may be referred to as an air filtration system. The air treatment system 100 may be at least one of: an air purifier, a dehumidifier, a humidifier and an air cooler. The air purifier may comprise, for example, ionization and filters. The dehumidifier may comprise for example mechanical and desiccant elements. The humidifier may comprise, for example, water vapor diffusion or ultrasonic misting. The air cooler may comprise, for example, evaporative cooling or hybrid systems. The purpose of the air treatment system 100 may be described as to apply surface charges to airborne particles to improve filtration efficiency.

The horizontal arrows pointing from the right to the left illustrates the air flow, i.e. the air input and output to and from the air treatment system 100.

Airborne particles may be electrostatically charged using flexible conductive elements upstream of a multilayer filter. The air treatment system 100 improves particle capture efficiency by combining active electrostatic charging with advanced filter materials, for example including graphene layers for enhanced antimicrobial performance and charge retention.

The air treatment system 100 may be located in a housing.

The air treatment system 100 may comprise a treatment part which may comprise at least one of a **pre-treatment unit 101** and a **primary treatment unit 103.** The input air may be first treated in the pre-treatment unit 101, and the air output by the pre-treatment unit 101 may thereafter be treated by the primary treatment unit 103. The input air may be treated only by the primary treatment unit 103, i.e. the input air is inputted directly into the primary treatment unit 103 without going via any pre-treatment unit 101.

The pre-treatment unit 101 may be arranged to pre-treat incoming air coming into the air treatment system 100, e.g. to condition incoming air. The pre-treatment unit 101 may be arranged to remove large particles or contaminants from the incoming air before it reaches the primary treatment unit, ensuring that the air is cleaner and reducing the burden on subsequent treatment processes. This leads to improved overall air quality. The pre-treatment unit 101 may be arranged to ensure consistent conditioning of incoming air before primary treatment stage.

The pre-treatment unit 101 comprises an electrostatic charging unit arranged to add electrostatic charging to the air. The electrostatic charging is added as a result of power provided from a power supply to the air treatment system 100. The electrostatic charging unit 101 may be arranged to be electrically connected to a power supply from which the electrostatic charging unit 101 obtains power. The power supply may provide, for example, pulsed or continuous voltage.

The reference number 101 is used herein when referring to both the pre-treatment unit and the electrostatic charging unit. The electrostatic charging unit 101 is arranged in an airstream of the air treatment system 100.

The electrostatic charging unit 101 comprising at least one **conductive element 104** arranged to apply surface charge to airborne particles in an airflow passing through the electrostatic charging unit 101. The at least one conductive element 104 may be arranged transverse to the direction of airflow through the air treatment system 100. In other words, the at least one conductive element 104 may be mounted across the airflow path. The at least one conductive element 104 may be mounted on a **support structure 108.** The support structure 108 is a fixed support structure. The support structure may be arranged in the direction of the airflow, i.e. The at least one conductive element 104 may be arranged in any suitable fashion to the support structure 108. The at least one conductive element 104 may be fixedly connected to the support structure 108 or it may be removably connected to the support structure 108.

The at least one conductive element 104 may be an at least partly flexible element. The at least one conductive element 104 may be at least one of: a brush-like element and a fiber-like element. The at least one conductive element 104 may be distributed across an area at least partly corresponding to a cross-section of an airflow channel. The airflow channel may be the channel through which the air flows through the air treatment system 100.

The air treatment system 100 may be modular and its components may be replaceable via a quick-connect interface. The quick-connect interface may be used to connect the air treatment components of the air treatment system 100, e.g. the electrostatic charging unit 101, the at least one sensor, to at least one of the other air treatment components of the air treatment system 100, to connect the air treatment components to a housing in which the air treatment system 100 is located. The quick-connect interface may be any suitable quick-connect interface, for example push-in terminals, blade or spade connectors, Molex-, JST- and Hirose connectors, Anderson Powerpole, WAGO 221/222 connectors, IDC connectors, Deutsch DT- and AMP superseal connectors, magnetic quick connectors, just to mention some examples. For example,
- Spring clamps, e.g. WAGO for connecting/removing the at least one sensor during service.
- Blade terminals for the power supply to the ionization module.
- Molex/JST connectors for plug-in connection of air quality sensors.
- IDC or WAGO push-in for integrating loT modules, e.g. Wi-Fi, BLE.
- Magnetic contact if parts should be easily removable/attachable with minimal effort, e.g., during cleaning.

The pre-treatment unit 101 may comprise an electrostatic ionizer for ionizing incoming air. The electrostatic ionizer may be arranged to charge airborne particles prior to filtration. Incoming air is ionized in the pre-treatment unit 101 prior to entering the mechanical filter of the primary treatment unit 103. For a humidifier, the pre-treatment unit 101 may comprise a pre-evaporation device. For a cooler, the pre-treatment unit 101 may comprise air chilling. One reason for doing the pre-treatment of the air may be that charged particles adhere more efficiently to the filter media and that it results in better filtration, longer air treatment component life, and even particle distribution.

The primary treatment unit 103 may be arranged to perform the main filtration or purification processes, effectively removing smaller particles, allergens, and harmful substances. The primary treatment unit 103 may be arranged to perform primary treatment of the air coming into the air treatment device 100. The primary treatment unit 103 may comprise at least one of: dehumidifying coil, humidifying chamber and cooling element.

The primary treatment unit 103 comprises an air filter through which the air passes and is filtered. The reference number 103 is used herein when referring to both the primary treatment unit and the air filter. The air filter 103 may be positioned downstream of the electrostatic charging unit 101. The air filter may be a multilayer filter comprising multiple air filters, e.g. at least two air filters. In other words, the air filter comprises *n* air filters, where *n* is a positive integer larger than one. The air filter 103 is arranged downstream of the electrostatic charging unit 101 arranged to filter the airflow from the electrostatic charging unit 101. The air filter 103 may be arranged transverse to the direction of airflow, i.e. in substantially the same direction as the at least one conductive element 104. In the case of a multiplayer filter, each filter may be connected to its neighbor filter using any suitable connecting means.

The downstream multilayer filter may be made of selectable materials. The air filter 103 may comprise at least two filter layers. Each of the at least two filter layers may be made of at least one of the following materials:
- polypropylene,
- polyester,
- acrylic fiber,
- polyamide,
- polyvinylidenefluoride, PVDF, nanofiber,
- Polytetrafluoroethylene, PTFE, fiber,
- carbon fiber, e.g. activated carbon,
- bamboo fiber,
- cellulose fiber,
- graphene-based material, e.g. graphene-enhanced antimicrobial layer, and
- photocatalytic coatings, e.g. photocatalytic TiO₂ layer.

In other words, the multilayer air filter 103 may comprise at least two layers selected from the group comprising: polypropylene, polyester, acrylic fiber, polyamide, PVDF nanofiber, PTFE, carbon fiber, bamboo fiber, cellulose fiber, graphene-based material, and photocatalytic coatings. In the case of a multilayer filter, all filters may be of the same or different material.

The at least two filter layers may comprise a combination of a graphene-based material with at least one of:
- carbon fiber
- photocatalytic TiO₂ coating, and
- polyvinylidenefluoride, PVDF nanofiber.

A layer may also be referred to as a coating.

Conventional air treatment systems typically rely on passive mechanical filtration or fixed ionizers to capture airborne particulates, limiting their efficiency under varying air conditions. These conventional systems may lack adaptability to variable air quality environments and are often limited in terms of material combinations. However, the air treatment system 100 comprising at least one of the materials listed above, for example graphene-based materials, along with dynamic control of charging intensity, may greatly improve both filtration performance and antimicrobial protection of the air treatment system 100. For example, graphene offer new opportunities for increased particle capture efficiency and improved hygiene.

The air treatment system 100 comprises at least one **sensor 105.** There may be *m* number of sensors, where *m* is a positive integer. The at least one sensor 105 may be any sensor arranged for sensing parameters relevant for the treatment of the air. The at least one sensor 105 may monitor factors such as pressure, airflow, temperature, humidity, and air quality, differential pressure, ionization intensity, pressure drop, optical recognition of filter color, saturation, particle levels, particle size, filter resistance and electric resistance. For example, the at least one sensor 105 may be a differential pressure sensor for measuring differential pressure across a filter medium. For example, the at least one sensor 105 may be an environmental sensor associated with an environment surrounding the air treatment system 100. For example, the at least one sensor 105 may be for example an optical, acoustic, or conductivity sensor for water/fan/mist status, for example in an air treatment device 100 such as a humidifier or cooler. For example, the at least one sensor 105 may be an air quality sensor arranged to measure the quality of the air at any suitable location inside and outside the air treatment system 100, for example before and after the pre-treatment unit 101, before and after the primary treatment unit 103, in relation to an air treatment component, just to mention some examples. The air quality sensor may be arranged to sense the air quality of the air between each filter in the multilayer filter 103.

The sensor 105 may be arranged to obtain real-time data. The real-time data from the at least one sensor 105 may enable dynamic adjustments to the air treatment processes based on current conditions, ensuring optimal performance and efficiency.

The at least one sensor 105 may be located at any suitable location inside or outside the air treatment system 100.

The air treatment system 100 may comprise or may be arranged to be connected to a **control unit 115.** In other words, the control unit 115 may be located within the air treatment system 100 or it may be an external control unit 115 arranged to communicate with the air treatment system 100. A first part of the control unit 115 may be comprised in the air treatment system 100 and a second part of the control unit 115 may be located offboard the air treatment system 100. The control unit 115 may be comprised in, for example, an external computer, a mobile phone, a tablet computer, a cloud computing unit, another air treatment device 100, etc. The control unit 115 comprises or is a computer system comprising a processing circuitry. The control unit 115 is arranged to control the air treatment system 100, for example to control the functioning of the air treatment system 100, to control the process of treating the air, to monitor the air treatment system 100, to control the electrostatic charge applied to the electrostatic charging unit 103, just to mention some examples. The control unit 115 is arranged to obtain measurements from the at least one sensor 105, e.g. the air quality sensor, and to process these measurements.

The control unit 115 may be arranged to control charging of the electrostatic charging unit 101 based on input from an air quality sensor 113. The control may comprise that, based on the input about the air quality obtained from the air quality sensor 113, the control unit 115 may analyze this information to determine if the air quality is below, at or above a threshold. When the air quality is below the threshold, the control unit 115 may determine that it may be an indication of that the air quality is bad or not sufficient, and that it may be necessary to change or increase the level of air treatment done by the air treatment system 100. The change or increase the level of air treatment may be done by that the control unit 115 may determine that an increase in the electrostatic charging unit 101 may be necessary in order to capture more airborne particles such as dust, pollen and other pollutants. The control unit 115 may send instructions to the power supply to increase its power supplied to the electrostatic charging unit 101, e.g. higher voltage, increased current, it may adjust any suitable settings that enables increased electrostatic charging, just to mention some examples.

When the air quality is at or above the threshold, the control unit 115 may determine that it may be an indication of that the air quality is good or sufficient, and that it may not be necessary to change or increase the level of air treatment done by the air treatment system 100 or that the air treatment may be stopped or paused.

The control unit 115 may be arranged to adjust the threshold, if necessary. The control unit may provide information indicating the analysis and result of the analysis to a user interface.

With the sensors 105 and the control unit 115 it may be possible to regulate the electrostatic charging process dynamically.

The control unit 115 may be arranged to determine an indicator indicative of at least one of:
- a need for maintenance of at least one air treatment system component,
- a need for replacement of at least one air treatment system component, e.g. the air filter 103, and
- an operational status of the at least one air treatment system component.

The control unit 115 may determine the indicator based on, for example, information obtained from the at least one sensor 105. The control unit 115 may analyze this information and the result may be at least one of the indicators listed above.

The control unit 115 may comprise smart or Al-driven logic for dynamic response to real-time sensor data, optimizing operational parameters for different environments and usage profiles. Thus, information from the at least one sensor 105 may be used by the control unit 115 in adaptive control of the air treatment system 100.

The control unit 115 comprises processing circuitry.

The control unit 115 may comprise a memory. The memory comprises one or more units to be used to store data on, such as indicator, sensor measurements, time information, parameters, thresholds, data and applications to perform the methods disclosed herein when being executed, and similar. The memory comprises instructions executable by the processing circuitry.

Furthermore, the control unit 115 may comprise a communication interface such as comprising a transmitter, a receiver, a transceiver and/or one or more antennas.

The air treatment system 100 may comprise or may be connected to a user interface. In other words, the user interface may be located within the air treatment system 100 or it may be an external user interface arranged to communicate with the air treatment system 100. The user interface may be comprised in the control unit 115. The user interface may be arranged to output user perceivable output. The user interface is arranged to obtain user input from a user to the air treatment system 100, and to provide user output from the air treatment system 100 to the user of the air treatment device 100. The user interface is arranged to handle any type of input and output, for example images, text, audio, tactile etc. The output may be, for example, an indicator determined by the control unit 115. The user interface may be a mobile or cloud-based interface for displaying maintenance status and predictions.

Air enters the air treatment system 100 and passes through the electrostatic charging unit 101, where particles receive surface charges via brush- or fiber-like elements. The charged air then moves through a modular multilayer air filter 103, where different layers sequentially capture coarse particles, VOCs, fine particles, and microbial agents. The control unit 115, coupled with an air quality sensor 105, dynamically regulates the charging intensity.

The present disclosure provides an adaptive air treatment system 100 with an upstream electrostatic charging unit 101 comprising conductive flexible elements, combined with a configurable multilayer air filter 103. The air filter 103 may comprise layers of any one or any combination of: polypropylene, polyester, PVDF nanofiber, PTFE, carbon fiber, bamboo fiber, cellulose, graphene-based materials, and photocatalytic coatings. The air treatment system 100 is designed for maximum flexibility, efficiency, and protection, including antimicrobial graphene-enhanced layers. The present disclosure enables superior particle capture with dynamic charging control. Furthermore, it provides enhanced microbial defense via graphene-based filter media. The air treatment system 100 comprises customizable filter stacks for different environments, e.g. home, office, industrial. The air treatment system 100 has an energy-efficient operation with real-time air quality adjustment. The air treatment system 100 is modular and comprises replaceable components for easy maintenance.

The present disclosure relates to an air treatment system 100 in which airborne particles are electrostatically charged via a brush- or fiber-based charging unit arranged upstream of a multilayer filter. The air filter 103 may comprise graphene-based materials to enhance charge retention, provide antimicrobial effects, and improve mechanical durability.

The embodiments herein are not limited to the above described embodiments. Various alternatives, modifications and equivalents may be used. Therefore, the above embodiments should not be taken as limiting the scope of the embodiments, which is defined by the appended claims. A feature from one embodiment may be combined with one or more features of any other embodiment.

The term "at least one of A and B" should be understood to mean "only A, only B, or both A and B.", where A and B are any parameter, number, indication used herein etc.

It should be emphasized that the term "comprises/comprising" when used in this specification is taken to specify the presence of stated features, integers, steps or components, but does not preclude the presence or addition of one or more other features, integers, steps, components or groups thereof. It should also be noted that the words "a" or "an" preceding an element do not exclude the presence of a plurality of such elements.

The term "configured to" used herein may also be referred to as "arranged to", "adapted to", "capable of" or "operative to".

It should also be emphasised that the steps of the methods defined in the appended claims may, without departing from the embodiments herein, be performed in another order than the order in which they appear in the claims.

## Claims

1. An air treatment system (100) comprising:
an electrostatic charging unit (101) comprising at least one conductive element (104) arranged to apply surface charge to airborne particles in an airflow passing through the electrostatic charging unit (101); and
an air filter (103) arranged downstream of the electrostatic charging unit (101) arranged to filter the airflow from the electrostatic charging unit (101).

2. The air treatment system (100) of any of the preceding claims, wherein the at least one conductive element (104) is arranged transverse to the direction of airflow through the air treatment system (100).

3. The air treatment system (100) of any of the preceding claims, wherein the at least one conductive element (104) is a flexible element.

4. The air treatment system (100) of any of the preceding claims, wherein the at least one conductive element (104) is at least one of: a brush-like element and a fiber-like element.

5. The air treatment system (100) of any of the preceding claims, wherein the electrostatic charging unit (101) is arranged to be electrically connected to a power supply from which the electrostatic charging unit (101) obtains power.

6. The air treatment system (100) of any of the preceding claims, wherein the at least one conductive element (104) is distributed across an area corresponding to a cross-section of an airflow channel.

7. The air treatment system (100) of any of the preceding claims, wherein the air filter (103) comprises at least two filter layers, wherein each of the at least two filter layers is made of at least one of, in any sequence or combination of:
• polypropylene,
• polyester,
• acrylic fiber,
• polyamide,
• polyvinylidenefluoride, PVDF, nanofiber,
• Polytetrafluoroethylene, PTFE, fiber,
• carbon fiber,
• bamboo fiber,
• cellulose fiber,
• graphene-based material, and
• photocatalytic coating.

8. The air treatment system (100) according to any of the preceding claims, wherein the air filter (103) comprises at least two filter layers, and wherein the at least two filter layers comprises a combination of a graphene-based material with at least one of:
• carbon fiber
• photocatalytic TiO₂ coating, and
• polyvinylidenefluoride, PVDF nanofiber.

9. The air treatment system (100) of any of the preceding claims, comprising or arranged to be connected to a control unit (115) arranged to control charging of the electrostatic charging unit (101) based on input from an air quality sensor (113).

10. The air treatment system (100) of claim 9, wherein the control unit (115) is arranged to determine an indicator indicative of at least one of:
• a need for maintenance of at least one air treatment system component,
• a need for replacement of the at least one air treatment system component, and
• an operational status of the at least one air treatment system component.

11. The air treatment system (100) of any of the preceding claims, wherein the air treatment system (100) is modular and its air treatment components are replaceable via a quick-connect interface.

12. The air treatment system (100) of any of the preceding claims, comprised in at least one of:
• an air purifier,
• a Heating, Ventilation and Air Conditioning, HVAC, system,
• an industrial air treatment system, and
• a healthcare air treatment system
